# EUROPEAN PATENT SPECIFICATION

(11) **EP 4 271 353 B1**
(45) Date of publication and mention of the grant of the patent: **17.07.2024**
(21) Application number: 21835730.9
(22) Date of filing: 13.12.2021
(51) Int. Cl.: A61K 8/34, A61K 8/41, A61Q 5/02, A61Q 5/12

(54) **HAIR CARE COMPOSITION**
HAARPFLEGEZUSAMMENSETZUNG
COMPOSITION DE SOINS CAPILLAIRES

(30) Priority: 30.12.2020 WO PCT/CN2020/141752; 05.02.2021 EP 21155346
(43) Date of publication of application: 08.11.2023
(73) Proprietor: Unilever IP Holdings B.V., 3013 AL Rotterdam (NL); Unilever Global IP Limited, Wirral, Merseyside CH62 4ZD (GB)
(72) Inventor: CAO, Qunhua, Shanghai 200335 (CN); JIN, Tong, Shanghai 200333 (CN); LIU, Jian, Shanghai 200335 (CN)
(74) Representative: Mathai, Neenu Grace
(86) International application number: PCT/EP2021/085337
(87) International publication number: WO 2022/144161

(56) References cited:
- WO-A1-2013/064365
- WO-A1-2018/053213
- WO-A1-2021/041760
- CN-A- 110 339 129
- JP-A- 2001 261 530
- JP-A- 2005 343 859
- JP-A- 2006 225 307
- JP-B2- 6 603 898

## Description

### Field of the Invention

The invention relates to a hair care composition. The invention particularly relates to a stable translucent hair care composition which exhibits the desired sensorial experience.

### Background of the Invention

Hair care compositions generally provide cleansing or conditioning benefits or a combination of the two. Hair cleansing compositions generally sold as shampoos typically comprise one or more anionic cleansing surfactants which generally aid in cleaning the hair and the scalp free of undesirable soil, particles and fatty matter. Hair conditioners are another class of hair care compositions which are generally used on hair in the wet condition after the hair has been washed with a shampoo. Compositions which provide the dual benefits of shampooing and conditioning are also known.

Hair conditioners generally comprise cationic surfactants. The conditioning benefit is achieved by including one or more conditioning agents in the hair care composition. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. The most popular hair conditioning agents used are silicones.

However, preferred hair care composition will provide more benefits than simple cleansing and conditioning. For example, it is desirable to incorporate various benefit agents in hair care composition to provide benefits in addition to cleansing and conditioning. For example, anti-dandruff benefit has been provided through hair care compositions. Dandruff is an issue that affects many people globally. The condition is manifested by the shedding of clumps of dead skin cells from the scalp. These are white in colour and provide an aesthetically displeasing appearance. A factor that contributes to dandruff is certain members of the Malassezia yeasts. To combat these, anti-dandruff products have included certain anti-dandruff agents which have anti-fungal activity, for example, piroctone olamine (Octopirox^{®}), zinc pyrithione, azole based anti-fungal agents (e.g. climbazole, ketoconazole), selenium sulfide or combinations thereof.

The present invention relates to a translucent hair care composition comprising a combination of a cationic surfactant, a fatty acid and glycerin. The present inventors have now found unexpectedly that the presence of certain level of glycerin and fatty acid can improve the transparency of the hair care composition while delivering the desired sensorial experience.

WO17127344 (P&G) relates to a hair conditioning composition comprising: (a) a cationic surfactant; (b) a high melting point fatty compound having a melting point of 25 degrees centigrade or higher; (c) a monoalkyl glyceryl ether; and (d) an aqueous carrier. The examples have 50% glycerin.

WO16115446 (P&G) relates to a hair conditioning composition comprising: a cationic surfactant; a high melting point fatty compound having a melting point of 25 degrees centigrade or higher; a material having a refractive index of from about 1.30 to about 1.70; and an aqueous carrier, wherein the composition has a transmittance of at least about 0.5 percent. The examples have 50% or 60% glycerin.

JP6603898 B2 discloses a pre-shampoo hair treatment composition comprising a cationic surfactant such stearamidopropyl dimethylamine, behentrimonium chloride or stearyl trimethyl ammonium chloride, 12-25% glycerine and 3-8% higher alcohol such as stearyl alcohol or cetyl alcohol.

### Summary of the Invention

In a first aspect, the present invention relates to a hair care composition comprising:
a) a cationic surfactant selected from stearamidopropyl dimethylamine, behentrimonium chloride, stearyl trimethyl ammonium chloride or mixtures thereof;
b) from 15 to 30% by weight of glycerin; and
c) from 0.1 to 2.0% by weight of fatty alcohol based on the total weight of the composition; wherein the composition comprises an antidandruff agent.

All other aspects of the present invention will more readily become apparent upon considering the detailed description and examples which follow.

### Detailed Description of the Invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. In other words, in specifying any ranges of values, any particular upper value can be associated with any particular lower value.

The disclosure of the invention, as found herein, is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

"Hair care composition", as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or bar. Non-limiting examples of such compositions include leave-on hair lotions, creams, and rinse-off shampoos, conditioners, shower gels, or toilet bar. The composition of the present invention is preferably a rinse-off composition, especially preferred being a shampoo or a conditioner and most preferably a conditioner.

The transparency of the hair care composition can be indicated by %transmittance. It is preferred that the %transmittance of the composition measured at wavelength of 600 nm is at least about 0.11 %, more preferably at least 0.12%, most preferably at least 0.13%.

### Cationic surfactant

The hair care composition of the present invention comprises a cationic surfactant selected from stearamidopropyl dimethylamine, behentrimonium chloride, stearyl trimethyl ammonium chloride or mixtures thereof.

The hair care composition typically comprises the cationic surfactant in an amount of from 0.1 to 4%, more preferably from 0.5 to 2%, based on total weight of the hair care composition and including all ranges subsumed therein.

### Fatty alcohol

Hair care composition of the present invention additionally comprise a fatty alcohol. The combined use of fatty alcohols and cationic surfactants is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. It is preferred that fatty alcohols include cetyl alcohol, stearyl alcohol and mixtures thereof. The use of these materials is also advantageous in that they contribute to the overall conditioning properties of compositions of the invention. However, the presence of high dose of fatty alcohol is believed to decrease the transparency of the hair care composition.

Therefore, the fatty alcohol present in the hair care composition of the present invention at a level ranging from 0.1 to 2%, preferably still from 0.2 to 2% and more preferably from 0.5 to 2%, furthermore preferably from 1 to 2% based on total weight of the hair care composition and including all ranges subsumed therein. The weight ratio of cationic surfactant to fatty alcohol is typically from 1:1 to 1:10, more preferably from 1:1.5 to 1:8, most preferably from 1:2 to 1:5.

### Glycerin

Glycerin for use in the present invention is variously known as glycerol, glycerine, and propane-1,2,3-triol.

The glycerin present in the hair care composition of the present invention at a level ranging from 15 to 30%, more preferably from 20 to 30%, furthermore preferably from 25 to 30%, and most preferably from 25 to 28%, based on total weight of the hair care composition and including all ranges subsumed therein.

### Antidandruff agent

The anti-dandruff agents present in the hair care composition are compounds that are active against dandruff and are typically anti-microbial agents and preferably anti-fungal agents. Suitable anti-dandruff agents that may be used in this invention are selected from piroctone olamine (Octopirox^{®}), selenium sulfide and mixtures thereof. In a preferred embodiment, the antidandruff agent is piroctone olamine.

Suitable anti-dandruff agents that may be used in this invention are selected from piroctone olamine (Octopirox^{®}), azole based anti-fungal agents, selenium sulfide and mixtures thereof. The preferred azole based anti-fungal agent is ketoconazole, climbazole or mixtures thereof. Preferably, the anti-dandruff agent is selected from piroctone olamine, climbazole and mixtures thereof. In an especially preferred embodiment, the anti-dandruff agent is piroctone olamine.

Typically, the hair care composition of the invention comprises the anti-dandruff agent in an amount of from 0.01 to 5%, more preferably from 0.01 to 3%, more preferably still from 0.05 to 2%, and most preferably from 0.05 to 1.5%, based on total weight of the hair care composition and including all ranges subsumed therein.

### Silicone

The composition of the present invention is targeted to compositions which are substantially free of silicone oils. By the phrase 'substantially free of silicone oils is meant that silicone oil is present in an amount less than 1.0%, preferably less than 0.5%, furthermore preferably less than 0.1% and optimally absent from the composition. It is most preferred that the composition is free of a silicone compound.

The silicone oil as per this invention is a silicone compound which is a non-volatile silicone. By non-volatile silicone is meant that the silicone has a vapor pressure less than 0.1 mm Hg (13.3 Pa), preferably less than 0.02 mm Hg, more preferably less than 0.01 mm Hg at 25 °C at one atmospheric pressure.

Silicone oils as per this invention include polydiorganosiloxanes, in particular polydimethylsiloxanes which have the CTFA designation dimethicone. Also included within this definition are polydimethyl siloxanes having hydroxyl end groups, which have the CTFA designation dimethiconol. Also included within the definition of silicone oils as per this invention are silicone gums having a slight degree of cross-linking, as are described for example in WO 96/31188 (UNILEVER).

Another class of silicones which is substantially excluded from the hair care compositions of the present invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

The hair care composition of the invention may comprise octyldodecanol. Octyldodecanol is a clear slightly yellow oily material. This is available under the brand name of Eutanol G from Cognis. It can be included in an amount of 0.01 to 5.0%, preferably 0.1 to 3% by weight of the composition.

Preservatives may also be incorporated into the hair care composition of this invention to protect against the growth of potentially harmful microorganisms. Suitable traditional preservatives include alkyl esters of parahydroxybenzoic acid, hydantoin derivatives, propionate salts, and a variety of quaternary ammonium compounds. Illustrative yet non-limiting examples of the types of preservatives that may be used in this invention include, for example, phenoxyethanol, sodium salicylate, methyl paraben, butyl paraben, propyl paraben, diazolidinyl urea, sodium dehydroacetate, 1,2-hexanediol, benzyl alcohol, sodium benzoate, iodopropynyl butylcarbamate, caprylyl glycol, disodium EDTA or mixtures thereof. In an especially preferred embodiment, the preservative is 1,2-hexanediol, benzyl alcohol or a mixture thereof. Preservatives are preferably employed in amounts ranging from 0.01 to 2% by weight of the hair care composition.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

The compositions of the invention are primarily intended for topical application to scalp and/or at least a portion of the hair of an individual, either in rinse-off or leave-on compositions, preferably in rinse-off compositions like conditioners.

The invention will now be described with reference to the following non-limiting examples.

### Examples

### Examples 1-5: Transparency improved by conditioner compositions comprising from 15 to 30% of glycerin.

The following hair care compositions as shown in Table 1 were prepared. The compositions were tested for their transparency by measuring the %transmittance at 600nm. The procedure used to measure the %transmittance is as given below:
Sample Preparation: The compositions were centrifuged to remove the error brought by bubbles. The equipment used was Sigma 3-18K Centrifuge with Rotor 19776. The parameter was set at 8000 rpm, 5minutes, 25°C.
%transmittance Test: %transmittance is the ratio of the intensity of the transmitted light (I) to the intensity if the incident light (I₀). The Equipment used was Cary Series UV-Vis Spectrophotometer in Total Transmittance Mode (TTRAN) mode with a transmission cell of 10 mm width. The % transmittance values of the samples were measured at wavelength of 600 nm.

**Table 1**

| **Components, wt%** | **ExA** | **ExB** | **ExC** | **Ex1** | **Ex2** | **Ex3** | **Ex4** | **Ex5** |
|---|---|---|---|---|---|---|---|---|
| Behentrimonium chloride | 1 | 1 | 1 | 1 | 1 | 1 | 1 | 1 |
| Cetearyl alcohol | 2 | 2 | 6 | 2 | 2 | 2 | 2 | 2 |
| Lactic Acid | 0.06 | 0.06 | 0.01 | 0.06 | 0.06 | 0.06 | 0.01 | 0.01 |
| Octopirox | 0.15 | 0.15 | | 0.15 | 0.15 | 0.15 | - | - |
| Climbazole | - | - | 0.25 | | - | - | 0.25 | 0.25 |
| Octyldodecanol | 0.5 | 0.5 | | 0.5 | 0.5 | 0.5 | - | - |
| Glycerin | 0 | 10 | 30 | 15 | 20 | 30 | 20 | 30 |
| Propylene Glycol | 1.2 | 1.2 | 0.5 | 1.2 | 1.2 | 1.2 | 0.5 | 0.5 |
| Water | To 100 | To 100 | To 100 | To 100 | To 100 | To 100 | To 100 | To 100 |
| % transmittance | 0.107 | 0.109 | 0.068 | 0.118 | 0.13 | 0.157 | 0.14 | 0.165 |

The data in Table 1 above indicates that compositions as per the invention (Examples 1-5) exhibits higher transparency as compared to compositions outside the invention (Examples A-C). It also indicated that the increase of amount of fatty alcohol (higher than 2%) will have negative impact on the transparency of the composition.

### Example 2: Sensory improved by conditioner compositions comprising from 15 to 30% of glycerin.

The following hair care composition as shown in Table 2 were prepared.

**Table 2**

| **Components, wt%** | **Ex2** | **ExD** |
|---|---|---|
| Behentrimonium chloride | 1 | 1 |
| Cetearyl alcohol | 2 | 2 |
| Lactic Acid | 0.06 | 0.06 |
| Octopirox | 0.15 | 0.15 |
| Octyldodecanol | 0.5 | 0.5 |
| Glycerin | 20 | 50 |
| Propylene Glycol | 1.2 | 1.2 |
| Water | To 100 | To 100 |

The sensorials delivered by the composition of the invention (Example 2) was compared to that of a composition outside the invention (Example D) in a Salon trial.

In the salon trial the samples were tested on 36 female respondents. The respondents were evaluated prior to the trial to have the same type of hair (e.g. normal hair, dry hair, greasy hair etc). In a given trial, 18 respondents with normal hair and 18 with greasy hair were chosen. The evaluation was done by trained hairdressers with good sensitivity. The evaluation method involved paired comparison with half the head washed with one sample and the other half of the head washed with the other sample.

The results showed that composition Example 2 was significantly (at least 95% conf.) higher in the following attributes as compared to Example D:
Wet Stage:
   - Longer time to rinse strip
Dry Stage:
   - Volume
   - More Visual Expansion from scalp
   - More shiny
   - Less sticky

The data from salon trial indicates that the compositions as per the invention (Example 2) has improved sensory when in use while exhibiting good transparency as compared to compositions outside the invention (Example D).

### Example 6: Transparency improved by conditioner compositions comprising low amount of fatty acid.

The following hair care compositions as shown in Table 3 were prepared. The compositions were tested for their transparency by measuring the %transmittance at 600nm. The procedure used to measure the %transmittance is same as what is disclosed above.

**Table 3**

| **Components, wt%** | **Ex E** | **Ex 6** |
|---|---|---|
| Behentrimonium chloride | 1 | 1 |
| Cetearyl alcohol | 3 | 2 |
| Lactic Acid | 0.01 | 0.01 |
| Climbazole | 0.25 | 0.25 |
| Glycerin | 30 | 30 |
| Propylene Glycol | 0.5 | 0.5 |
| Water | To 100 | To 100 |
| % transmittance | 0.116 | 0.181 |

The data in Table 3 above indicates that compositions as per the invention (Example 6) exhibits higher transparency as compared to composition outside the invention (Example E). It also indicates that the increase of amount of fatty alcohol (higher than 2%) will have negative impact on the transparency of the composition.

## Claims

1. A translucent hair care composition comprising:
a) a cationic surfactant selected from stearamidopropyl dimethylamine, behentrimonium chloride, stearyl trimethyl ammonium chloride or mixtures thereof;
b) from 15 to 30% by weight of glycerin; and
c) from 0.1 to 2.0% by weight of fatty alcohol based on the total weight of the composition; wherein the composition comprises an antidandruff agent.

2. A composition as claimed in claim 1 wherein the antidandruff agent is selected from piroctone olamine, selenium sulfide, azole based anti-fungal agents and mixtures thereof.

3. A composition as claimed in claim 2 wherein said antidandruff agent is piroctone olamine, climbazole or mixture thereof.

4. A composition as claimed in claims 2 or 3 wherein the antidandruff agent is present in an amount of 0.01 to 5% by weight of the composition.

5. A composition as claimed in any of claims 1 to 4 wherein the cationic surfactant is behentrimonium chloride.

6. A composition as claimed in any of claims 1 to 5 wherein the cationic surfactant is present in an amount of from 0.01 to 4.0% by weight of the composition.

7. A composition as claimed in any of claims 1 to 6 wherein said fatty alcohol comprises from 8 to 22 carbon atoms.

8. A composition as claimed in claim 7 wherein said fatty alcohol comprises from 16 to 22 carbon atoms.

9. A composition as claimed in any of claims 1 to 8 wherein the fatty alcohol is selected from the group consisting of cetyl alcohol, stearyl alcohol, and mixtures thereof.

10. A composition as claimed in any of claims 1 to 9 wherein the fatty alcohol is present in an amount of from 0.5 to 2.0% by weight of the composition.

11. A composition as claimed in any of claims 1 to 10 wherein the weight ratio of cationic surfactant to fatty alcohol is from 1:1 to 1:5.

12. A composition as claimed in any of claims 1 to 11 wherein said composition comprises less than 1% silicone compound, preferably less than 0.1% silicone compound by weight of the composition.

13. A composition as claimed in any of claims 1 to 12 wherein said composition is free of a silicone compound.

14. A composition as claimed in any of claims 1 to 13 wherein said composition comprises from 0.01 to 5.0% by weight of octyldodecanol.

## Patentansprüche

1. Durchscheinende Haarpflegezusammensetzung, umfassend:
a) ein kationisches Tensid, ausgewählt unter Stearamidopropyldimethylamin, Behentrimoniumchlorid, Stearyltrimethylammoniumchlorid oder Mischungen davon;
b) 15 bis 30 Gewichts-% Glycerin; und
c) 0,1 bis 2,0 Gewichts-% Fettalkohol, bezogen auf das Gesamtgewicht der Zusammensetzung; wobei die Zusammensetzung ein Antischuppenmittel umfasst.

2. Zusammensetzung, wie im Anspruch 1 beansprucht, wobei das Antischuppenmittel unter Piroctonolamin, Selensulfid, Antimykotika auf Azol-Basis und Mischungen davon ausgewählt ist.

3. Zusammensetzung, wie im Anspruch 2 beansprucht, wobei das Antischuppenmittel Piroctonolamin, Climbazol oder eine Mischung davon ist.

4. Zusammensetzung, wie in den Ansprüchen 2 oder 3 beansprucht, wobei das Antischuppenmittel in einer Menge von 0,01 bis 5 Gewichts-% der Zusammensetzung vorliegt.

5. Zusammensetzung, wie in einem der Ansprüche 1 bis 4 beansprucht, wobei das kationische Tensid Behentrimoniumchlorid ist.

6. Zusammensetzung, wie in einem der Ansprüche 1 bis 5 beansprucht, wobei das kationische Tensid in einer Menge von 0,01 bis 4,0 Gewicht-% der Zusammensetzung vorliegt.

7. Zusammensetzung, wie in einem der Ansprüche 1 bis 6 beansprucht, wobei der Fettalkohol 8 bis 22 Kohlenstoffatome umfasst.

8. Zusammensetzung, wie im Anspruch 7 beansprucht, wobei der Fettalkohol 16 bis 22 Kohlenstoffatome umfasst.

9. Zusammensetzung, wie in einem der Ansprüche 1 bis 8 beansprucht, wobei der Fettalkohol aus der Gruppe, bestehend aus Cetylalkohol, Stearylalkohol und Mischungen davon, ausgewählt ist.

10. Zusammensetzung, wie in einem der Ansprüche 1 bis 9 beansprucht, wobei der Fettalkohol in einer Menge von 0,5 bis 2,0 Gewichts-% der Zusammensetzung vorliegt.

11. Zusammensetzung, wie in einem der Ansprüche 1 bis 10 beansprucht, wobei das Gewichtsverhältnis des kationischen Tensids zum Fettalkohol 1:1 bis 1:5 beträgt.

12. Zusammensetzung, wie in einem der Ansprüche 1 bis 11 beansprucht, wobei die Zusammensetzung weniger als 1% Silikonverbindung, bevorzugt weniger als 0,1% Silikonverbindung, bezogen auf das Gewicht der Zusammensetzung, umfasst.

13. Zusammensetzung, wie in einem der Ansprüche 1 bis 12 beansprucht, wobei die Zusammensetzung frei von einer Silikonverbindung ist.

14. Zusammensetzung, wie in einem der Ansprüche 1 bis 13 beansprucht, wobei die Zusammensetzung 0,01 bis 5,0 Gewichts-% Octyldodecanol umfasst.

## Revendications

1. Composition de soin capillaire translucide comprenant :
a) un tensioactif cationique choisi parmi la stéaramidopropyl diméthylamine, le chlorure de béhentrimonium, le chlorure de stéaryl triméthyl ammonium ou les mélanges de ceux-ci ;
b) de 15 à 30 % en poids de glycérine ; et
c) de 0,1 à 2,0 % en poids d'alcool gras sur la base du poids total de la composition ; dans laquelle la composition comprend un agent antipelliculaire.

2. Composition selon la revendication 1 dans laquelle l'agent antipelliculaire est choisi parmi la piroctone olamine, le sulfure de sélénium, les agents antifongiques à base d'azote et les mélanges de ceux-ci.

3. Composition selon la revendication 2 dans laquelle ledit agent antipelliculaire est de la piroctone olamine, du climbazole ou un mélange de ceux-ci.

4. Composition selon les revendications 2 ou 3 dans laquelle l'agent antipelliculaire est présent en une quantité de 0,01 à 5 % en poids de la composition.

5. Composition selon l'une quelconque des revendications 1 à 4 dans laquelle le tensioactif cationique est du chlorure de béhentrimonium.

6. Composition selon l'une quelconque des revendications 1 à 5 dans laquelle le tensioactif cationique est présent en une quantité de 0,01 à 4,0 % en poids de la composition.

7. Composition selon l'une quelconque des revendications 1 à 6 dans laquelle ledit alcool gras comprend de 8 à 22 atomes de carbone.

8. Composition selon la revendication 7 dans laquelle ledit alcool gras comprend de 16 à 22 atomes de carbone.

9. Composition selon l'une quelconque des revendications 1 à 8 dans laquelle l'alcool gras est choisi dans le groupe constitué d'alcool cétylique, d'alcool stéarylique, et de mélanges de ceux-ci.

10. Composition selon l'une quelconque des revendications 1 à 9 dans laquelle l'alcool gras est présent en une quantité de 0,5 à 2,0 % en poids de la composition.

11. Composition selon l'une quelconque des revendications 1 à 10 dans laquelle le rapport pondéral du tensioactif cationique à l'alcool gras est de 1:1 à 1:5.

12. Composition selon l'une quelconque des revendications 1 à 11 dans laquelle ladite composition comprend moins de 1 % de composé de silicone, de préférence moins de 0,1 % de composé de silicone en poids de la composition.

13. Composition selon l'une quelconque des revendications 1 à 12 dans laquelle ladite composition est exempte d'un composé de silicone.

14. Composition selon l'une quelconque des revendications 1 à 13 dans laquelle ladite composition comprend de 0,01 à 5,0 % en poids d'octyldodécanol.
